# EUROPEAN PATENT SPECIFICATION

(11) **EP 0 352 250 B1**
(45) Date of publication and mention of the grant of the patent: **11.09.1996**
(21) Application number: 89870113.1
(22) Date of filing: 20.07.1989
(51) Int. Cl.: C12N 15/31, C12P 21/00, A61K 39/10

(54) **Bordetella pertussis vaccine**
Impfstoff gegen Bordetella pertussis
Vaccin contre Bordetella pertussis

(30) Priority: 22.07.1988 US 222991
(43) Date of publication of application: 24.01.1990
(73) Proprietor: SMITHKLINE BEECHAM BIOLOGICALS s.a., B-1330 Rixensart (BE)
(72) Inventor: Capiau, Carine, B-7092 Mons (BE); Locht, Camille, B-1320 Genval (Rixensart) (BE)
(74) Representative: Tyrrell, Arthur William Russell, Dr.

(56) References cited:
- EP-A- 396 964
- EP-A- 0 232 229
- EP-A- 0 322 115
- EP-A- 0 322 533
- WO-A-87/07507
- Infection and Immunity, vol. 56, no 8, August 1988; Joseph T. BARBIERI ET AL.: pp. 1934-1941
- Proc. Natl. Acad. Sci. USA; vol. 86, May 1989, Washington US; CAMILLE LOCHT et al.: pp. 3075-3079
- JOURNAL OF CELL BIOLOGY, vol. 107, no. 6 part.3, 1988, US; S. LOOSMORE etal.: page 420A
- INFECTION AND IMMUNITY vol. 55, no. 10, October 1987; William J. Black et al.: pp. 2465-2470
- JOURNAL OF BIOLOGICAL CHEMISTRY, vol. 264, no. 29, 15 October 1989, Baltimore, US; Galen CORTINA et al. pp. 17322-17328
- BIOLOGICAL ABSTRACTS DATABANK; abstract no. 88088941, J.T. BARBIERI et al.: & J. BACHTERIOLOGY 1989, vol. 171, no. 8, pp. 4362-4369

## Description

### Field of the Invention

This invention relates to a vaccine for protection against whooping cough which comprises protein encoded by a B. pertussis toxin coding sequence containing an entire or truncated B. pertussis toxin S1 subunit amino acid coding sequence which contains a modification of the tryptophan residue at amino acid position 26.

### Background of the Invention

Whooping Cough, or pertussis, is a highly infectious disease which primarily affects children. In addition to causing respiratory complications, whooping cough may result in nerve damage and high mortality, particularly in children in low socioeconomic groups and in newborn infants without maternal anti-pertussis antibodies. The etiologic agent of pertussis is the gram negative coccobacillus known as Bordetella pertussis. The bacteria is believed to invade the respiratory tract and induce a toxic state which remains even after the disappearance of the bacteria.

There remains a continuing need for effective and safe vaccines against infection caused by B. pertussis.

### SUMMARY OF THE INVENTION

The present invention relates to a B. pertussis toxin coding sequence containing an entire or truncated B. pertussis toxin S1 subunit amino acid coding sequence provided that (a) the S1 subunit coding sequence contains a modification of the tryptophan residue at amino acid position 26 (hereinafter "Trp-26"), and (b) the Trp-26 modification results in substantially inactivated S1 enzymatic activity in the protein encoded by the B. pertussis toxin coding sequence but such protein has retained the capacity to be recognized by anti-pertussis toxin antibodies. This invention also relates to a recombinant DNA molecule containing the coding sequence of this invention.

As a further embodiment of the invention, there is provided a recombinant DNA plasmid comprising the coding sequence of this invention. This plasmid preferably contains the above coding sequence in operative association with suitable expression control sequences. By 'suitable expression control sequences' is meant those sequences which are required to enable expression of the coding sequence of this invention by a suitable host cell into which the plasmid of this invention is introduced. Such expression control sequences are well known in the art.

Another aspect of the invention is a method of producing a transformed host cell which comprises transforming a desired host cell with the plasmid of the invention.

Still a further aspect of this invention is a host cell transformed with the plasmid of this invention. Such host cell is capable of growth in a suitable culture medium and expressing the coding sequence of this invention.

Yet a further aspect of the invention involves a method for producing the protein of the invention which comprises culturing the transformed host of the invention in a suitable culture medium and isolating the protein so produced. The present invention also relates to the protein encoded by the coding sequence of this invention.

Another embodiment of the invention is a vaccine for stimulating protection-against whooping cough, wherein such vaccine comprises an immunoprotective and non-toxic amount of the protein of the invention. Such vaccine may comprise such protein alone or in conjunction with other antigens and/or adjuvants. By the term 'immunoprotective amount' is meant an amount of any protein of this invention which will elicit a sufficient protective immune response against pertussis after such amount of such protein is administered to a human host.

In addition, the present invention also provides a method for inoculating a human against whooping cough which comprises administering the vaccine of this invention to such human.

Another embodiment of this invention is a process for producing the protein encoded by a Bordetella pertussis toxin amino acid coding sequence containing an entire or truncated B. pertussis toxin S1 subunit amino acid coding sequence provided that (a) the S1 subunit coding sequence contains a modification of the tryptophan residue at amino acid position 26 (hereinafter "Trp-26"), and (b) the Trp-26 modification results in substantially inactivated S1 enzymatic activity in the protein encoded by the B. pertussis toxin operon amino acid coding sequence but such protein has retained the capacity to be recognized by anti-pertussis toxin antibodies, wherein such process comprises (i) treating a Bordetella pertussis toxin amino acid coding sequence, prepared by isolation from native B. pertussis DNA or by synthetic or recombinant DNA techniques, containing an entire or truncated B*.* pertussis toxin S1 subunit amino acid coding sequence, provided that the S1 subunit coding sequence does not already contain a modification of the tryptophan residue at amino acid position 26, with site-directed mutagenesis, a tryptophan modifying chemical reagent or photooxidation to produce a modification or deletion of the tryptophan residue at amino acid position 26, and transforming a desired host cell with a plasmid containing such resulting coding sequence and culturing such host in suitable culture medium and isolating the protein so produced, (ii) substituting or deleting the tryptophan residue at position 26 of the S1 subunit of a Bordetella pertussis toxin amino acid coding sequence, prepared by isolation from native B. pertussis DNA or by synthetic or recombinant DNA techniques, containing an entire or truncated B. pertussis toxin S1 subunit amino acid coding sequence with another amino acid, and transforming a desired host cell with a plasmid containing such resulting coding sequence and culturing such host in suitable culture medium and isolating the protein so produced, or (iii) preparing such coding sequence by synthetic methods and transforming a desired host cell with a plasmid containing such coding sequence and culturing such host in suitable culture medium and isolating the protein so produced. This invention also relates to the protein produced by such process, a vaccine for stimulating protection against whooping cough wherein such vaccine comprises an immunoprotective and non-toxic quantity of such protein, and a method for innoculating a human against subsequent whooping cough which comprises administering the vaccine to such human.

A further aspect of this invention is a B. pertussis strain in which the chromosomal pertussis toxin gene is inactivated, wherein such strain contains the recombinant DNA molecule of this invention. Another embodiment of the invention is a whole cell vaccine for stimulating protection against whooping cough, wherein such vaccine comprises an immunoprotective and non-toxic amount of the strain of the invention. Such vaccine may comprise such strain alone or in conjunction with other antigens and/or adjuvants. Still another aspect of this invention relates to a method for innoculating a human against subsequent whooping cough which comprises administering the vaccine to such human.

Other Aspects and advantages of the present invention will be disclosed in the following detailed description of the presently preferred embodiment thereof.

### PRIOR ART

European Patent Application EP-A-0 396 964 (hereafter Document D1) discloses Bordetella toxin mutants having, inter alia, at least one of the residues, Trp 26, Glu 129 and Arg 9, substituted by a different amino acid. A specific mutant designated PT-261/129G is disclosed in which the Trp 26 and Glu 129 residues of the S1 subunit have been replaced by Ile and Gly respectively. This citation is relevant to the present application under Article 54(3) EPC for all designated states.

C. Locht *et al,* Proc Natl Acad Sci USA Vol 86 (May 1989), pages 3075-3079 (hereafter document D2) discloses experiments in which the S1 subunit of pertussis toxin is modified by deletion or substitution at three amino acid residues, Trp 26, Glu 106 and Glu 129. The modifications at Trp 26 and Glu 129 were shown to affect enzyme activity. The abstract of D2 states that pertussis toxin containing a deletion or replacement of Trp 26, Glu 129 or both in the S1 subunit should be devoid of toxic activities without losing reactivity with protective antibodies.

In the light of documents D1 and D2, the present invention is limited in accordance with the appended claims so that if, in addition to the modification to the tryptophan residue at position 26, there is additionally comprised a modification of a further amino acid residue in the S1 coding sequence then the additional modification does not include deletion or substitution of the the glutamic acid residue at position 129 and that when the tryptophan residue at position 26 of the S1 subunit coding sequence is substituted by isoleucine, the additional modification does not include substitution of the aspartic acid residue at amino acid position 11 by serine, substitution of the arginine residue at amino acid position 9 by lysine or glycine, substitution of the phenylalanine residue at position 50 by glutamic acid, substitution of the arginine residue at position 13 by leucine, substitution of the aspartic acid residue at position 1 by glutamic acid, substitution of the glutamic acid residue at position 39 by glycine, substitution of the tyrosine residue at position 130 by glycine, substitution of the glycine residue at position 86 by glutamic acid, substitution of the isoleucine residue at position 88 by glutamic acid, substitution of the tyrosine residue at position 89 by serine, substitution of the tyrosine residue at position 8 by aspartic acid or substitution of the threonine residue at position 53 by isoleucine.

These limitations apply to all 'double mutants' mentioned hereinbelow.

### DETAILED DESCRIPTION OF THE INVENTION

The present invention is based on the the discovery that modification of the naturally occurring tryptophan amino acid residue at position 26 of the S1 subunit of the pertussis toxin coding sequence greatly reduces the toxicity of pertussis toxin while retaining its ability to elicit a protective immunogenic response.

Pertussis toxin is a protein exotoxin of B. pertussis which plays a major role in the pathogenesis of whooping cough and is believed to be the major protective antigen of B. pertussis. [See, A.A. Weiss et al., Ann. Rev. Microbiol., 40:661 (1986)]. Pertussis toxin is a hexameric protein composed of five dissimilar subunits called S1 through S5 according to their decreasing molecular weights. [See, M. Tamura et al., Biochem., 21:5516 (1982)]. Pertussis toxin is structured in an A-B model in which the B-oligomer (subunits S2 through S5) is responsible for the binding of the toxin to its receptor in the target cell membranes, and the A-protomer (S1 subunit) contains enzymatic activity.

It has now been found that due to the Trp-26 modification of the S1 subunit contained by the coding sequence of this invention, the coding sequence encodes a protein which has substantially inactivated S1 subunit enzymatic activity. The normal S1 subunit enzymatic activity contributes to the toxicity of a variety-of B. pertussis vaccines-containing pertussis toxin or S1 subunit protein. Such enzymatic activity includes NAD-glycohydrolysis or ADP-ribosyltransfer. Thus, the vaccine of this invention has no such toxicity problems. Additionally, such inactivation of the S1 subunit enzymatic activity nevertheless allows the protein derived from the coding sequence of this invention to retain recognition by anti-pertussis toxin antibodies.

The present invention thus describes a B. pertussis toxin amino acid coding sequence containing an entire or truncated B. pertussis toxin S1 subunit amino acid coding sequence provided that (a) the S1 subunit coding sequence contains a modification of the tryptophan residue at amino acid position 26 (hereinafter "Trp-26"), and (b) the Trp-26 modification results in substantially inactivated S1 subunit enzymatic activity in the protein encoded by the B. pertussis toxin coding sequence but such protein retains the capacity to be recognized by anti-pertussis toxin antibodies. This invention also describes a recombinant DNA molecule comprising the coding sequence of this invention in addition to the B. pertussis toxin amino acid coding sequence of this invention; the recombinant DNA molecule of the invention may comprise additional DNA sequences, including, e.g., a regulatory element, one or more selection markers, and replication and maintainance functions.

By the term "B. pertussis toxin coding sequence" as used in the description of the coding sequence of this invention is meant the entire B. pertussis toxin operon or any functional truncated fragment thereof which contains an entire or truncated B. pertussis toxin S1 subunit amino acid coding sequence provided that (a) the S1 subunit coding sequence contains a modification at Trp-26, and (b), the Trp-26 modification results in substantially inactivated S1 subunit enzymatic activity in the protein encoded by the B. pertussis toxin coding sequence but such protein retains the capacity to be recognized by anti-pertussis toxin antibodies. By the term "B. pertussis toxin coding sequence" as used in the description of the coding sequence of this invention is also meant any functional fusion protein coding sequence comprising (i) the entire B. pertussis toxin coding sequence or any functional truncated fragment thereof which contains an entire or truncated B. pertussis toxin S1 subunit coding sequence, and (ii) any other protein coding sequence, provided that (a) the S1 subunit coding sequence contains a modification at Trp-26, and (b) the Trp-26 modification results in substantially inactivated S1 subunit enzymatic activity in the protein encoded by the B. pertussis toxin coding sequence but such protein retains the capacity to be recognized by anti-pertussis toxin antibodies. By the term "functional" is meant any B. pertussis toxin coding sequence which encodes a protein which will either be sufficiently recognizable by anti-pertussis toxin antibodies to be useful in a diagnostic assay for B. pertussis, and/or will elicit a sufficient protective immune response against pertussis after an immunoprotective amount of such protein is administered to a human host.

Preferably, the B. pertussis toxin coding sequence of this invention is a truncated version of the B. pertussis toxin operon comprising only the B. pertussis toxin S1 subunit amino acid coding sequence or a truncated portion thereof. Other preferred embodiments of the B. pertussis toxin amino acid coding sequence of this invention include the complete B. pertussis operon, or the coding sequence of any subunit thereof either alone or in conjunction with one or several other subunits.

The nucleotide coding-sequence of the entire pertussis toxin operon has been determined. See, e.g., Keith, et al., United States Patent Application Serial No. (U.S.S.N.) 843,727, filed March 26, 1986 (US 4,883,761). Furthermore, DNA containing the pertussis toxin coding sequence may be isolated from any publicly available B. pertussis strain. For example, various strains of B. pertussis are publicly available from commercial depositories, e.g., from the American Type Culture Collection, Rockville, Maryland, U.S.A. Such isolation may be effected by the method of Keith, et al., U.S.S.N. 843,727, supra, or any other conventional method.

Modification of the Trp-26 residue of the S1 subunit portion of the coding sequence of this invention may be effected in a variety of ways utilizing conventional techniques.

For example, one method of alteration of the Trp-26 residue is by use of a tryptophan (Trp) modifying chemical reagent such as 2-hydroxy-5-nitrobenzyl bromide (HNBB). In one embodiment of the invention, the catalytic domain for the NAD-glycohydrolase and ADP-ribosyltransferase activities of the pertussis toxin S1 subunit, e.g., amino acid residues 2-187 of the S1 subunit, was purified from an E. coli strain that expresses the gene coding for this catalytic domain. This purified polypeptide was treated with a tryptophan modifying chemical reagent (HNBB) to produce a modification of the Trp-26 residue. When the polypeptide resulting from the HNBB treatment was subsequently assayed for its S1 enzymatic activities, it was determined that it had no such activities, i.e., both NAD-glycohydrolase and ADP-ribosyltransferase activities were abolished as measured by standard assay procedures.

Additional tryptophan modifying chemical reagents which can be employed to effect modification of the Trp-26 residue include, but are not limited to, N-Bromosuccinimide (See, e.g., Spande, T.F. & Withop, B., 1967, Meth. Enzymol., 11:522), H₂O₂, performic acid, sulfenyl halides (See, e.g., Seolfone, E., Fontana, A. & Roccki, R., 1968, Biochemistry, 7:971), 2-nitrophenylsulfenyl chloride, 2,4-dinitrophenyl-sulfenyl chloride, and 2-acetoxy-5 nitrobenzyl chloride.

Photooxidation may also be employed as a method for modifying the Trp-26 residue of the S1 subunit. (See, e.g., Spikes, J.D. & Straight, R., 1967, Ann. Rev. Phys. Chem. 18:409),

Yet another means of modifying the Trp-26 residue of the S1 subunit includes the deletion or substitution of the codon for that residue in the gene coding for S1 subunit by conventional site-directed mutagenesis. One example of the performance of site directed mutagenesis on an exemplary construct of the coding sequence of this invention in the form of an S1 subunit gene is described in Example 5. For example, the S1 subunit coding sequence was subcloned into an M13 vector so that expression of this sequence could be produced in E. coli transformed by the recombinant M13 vector. The Trp-26 residue was then deleted, or changed to threonine (Thr), by site directed mutagenesis. Other vector constructs of the invention may be conventionally designed by one of skill in the art to perform such site-directed mutagenesis at the Trp-26 site.

An alternative method for modification of the tryptophan residue at position 26 of the S1 subunit coding sequence to create the coding sequence of the invention includes the deletion of the Trp-26 residue and its substitution by threonine (Thr) by conventional genetic engineering techniques or substitution at that site by other suitable amino acid residues. Other suitable substitute amino acids include, but are not limited to, glutamine (Gln), tyrosine (Tyr) and phenylalanine (Phe). A Trp-26 to Gln change is a more conservative replacement in terms of secondary structure propensity. A Trp to Tyr change may maintain an intermediate polarity characteristic, while a Trp to Phe change maintains the aromatic ring. The modified molecule should resemble the natural molecule as much as possible, therefore one would like a very similar secondary structure, hydrophobicity profile and proportion of aromatic amino acids. Any other amino acid substitutions may be considered provided that the substitution results in the creation of a coding sequence encoding a protein with substantially inactivated S1 enzymatic activity and retention of the anti-B. pertussis toxin antibody recognition.

The amino acid coding sequence of this invention may also be produced (a) synthetically, using conventional DNA synthesis techniques, or (b) by other chemical modification methods using conventional chemical modification techniques.

Additionally, by employing conventional genetic engineering techniques, the coding sequence of this invention may be in the form of any truncated version of the pertussis toxin operon containing the Trp-26 modification, or in the form of a truncated version of the S1 subunit coding sequence containing only the codons for amino acids 2 through 187 of the S1 subunit coding sequence including the Trp-26 modification, or alternatively, the coding sequence of this invention may contain any truncated portion of the S1 subunit coding sequence including the Trp-26 modification.

Furthermore, the coding sequence of this invention may be in the form of a double mutant, i.e., a coding sequence containing both a Trp-26 modification as well as a modification of another amino acid residue in the S1 subunit provided that the protein encoded by such coding sequence retains the capacity to be recognized by anti-pertussis toxin antibodies. It will be understood that such 'double mutants' falling within the scope of the invention are limited in view of the prior art (see Prior Art Section here-above and the appended claims). Preferably, the protein encoded by such coding sequence also exhibits a decrease in S1 subunit enzymatic activity. Examples of such additional modifications which may be employed in appropriate cases include, but are not limited to modification of the histidine residue at amino acid position 35 of the S1 subunit coding sequence (His-35); modification of the arginine residue at amino acid position 9 of the S1 subunit coding sequence (Arg-9) and modification of the serine residue at amino acid position 40 of the S1 subunit coding sequence (Ser-40).

Moreover, the coding sequence of the invention may appear in the form of a fusion protein coding sequence comprising the B. pertussis toxin amino acid coding sequence fused to at least one other coding sequence, such as, but not limited to, a pertussis toxin derived coding sequence and/or a non-pertussis toxin derived coding sequence. For example, such fusion protein may comprise all or part of the S1 subunit coding sequence including the Trp-26 modification fused to all or a portion of the S2 subunit coding sequence and/or the S3 subunit coding sequence and/or the S4 subunit coding sequence and/or the S5 subunit coding sequence. Such fusion protein may also comprise all or part of the S1 subunit coding sequence including the Trp-26 modification fused to other antigens of B. pertussis or other antigens such as, but not limited to, Diphtheria antigens and/or Tetanus antigens and/or Haemophilus influenzae antigens. An example of a fusion protein coding sequence of this invention is the one described in the examples which contains the following sequence:

### Sequence:

ATG   ACC  ATG  ATT ACG AAT TCG  AGC  TCG  GTA  CCC GGG GAT
(Met) Thr  Met  Ile Thr Asn Ser  Ser  Ser  Val  Pro Gly Asp
      beta-galactosidase        polylinker              2
                                                        S1

CCT  CCC  GCC  ACC  GTA......GTA  GCG  TCG  ATC  CTC  TAG
Pro  Pro  Ala  Thr  Val......Val  Ala  Ser  Ile  Leu  Stop
 3    4    5    6    7       184  185  186  187  polylinker
           S1

Other coding sequences of this invention which are in the form of a fusion protein can be conventionally designed and prepared by one of skill in the art.

Furthermore, the above Trp-26 modified S1 truncated fragment can be associated with other sequences of the wild-type pertussis toxin operon such as any other of the S2-S5 subunit coding sequences alone or in conjunction with one or more of the S2 through S5 subunits coding sequences. Alternatively, a complete B. pertussis toxin coding sequence may be generated containing (a) the above Trp-26 modified S1 truncated fragment replacing the naturally occurring S1 cistron or, (b) a complete S1 subunit cistron containing only the Trp 26 modification or some other fragment containing such a Trp-26 modification in association with other components.

Still a further aspect of this invention is a host cell transformed with the plasmid of this invention. Such host cell is capable of growth in a suitable culture medium and expressing the coding sequence of the invention. Such host cell is prepared by the method of this invention, i.e., transforming a desired host cell with the plasmid of the invention. Such transformation is accomplished by utilizing conventional transformation techniques. The most preferred host cells include those belonging to the species E. coli. and the genus Bordetella. Other host cells which may be suitable include, but are not limited to, mammalian cells, insect cells, other bacterial cells and yeast cells. Thus, this invention is not limited to any specific host cells.

The present invention also relates to a method of producing the protein encoded by the coding sequence of this invention which comprises culturing the transformed host of the invention in appropriate culture media and isolating such protein. By "appropriate culture media" is meant that media which will enabled the transformed host to grow and which will also enable such host to express the coding sequence of the invention in recoverable quantity. It will be appreciated by one of skill in the art that the appropriate culture media to use will depend upon the host cell employed. The isolation of the protein so produced is accomplished from a culture lysate of the host or, if appropriate, directly from the host's culture medium, and is carried out by conventional protein isolation techniques.

Thus, in view of the above description, another embodiment of this invention is a process for producing the protein encoded by a Bordetella pertussis toxin amino acid coding sequence containing an entire or truncated B. pertussis toxin S1 subunit amino acid coding sequence provided that (a) the S1 subunit coding sequence contains a modification of the tryptophan residue at amino acid position 26 (hereinafter "Trp-26"), and (b) the Trp-26 modification results in substantially inactivated S1 enzymatic activity in the protein encoded by the B. pertussis toxin operon amino acid coding sequence but such protein has retained the capacity to be recognized by anti-pertussis toxin antibodies, wherein such process comprises (i) treating a Bordetella pertussis toxin amino acid coding sequence, prepared by isolation from native B. pertussis DNA or by synthetic or recombinant DNA techniques, containingan entire or truncated B. pertussis toxin S1 subunit amino acid coding sequence, provided that the S1 subunit coding sequence does not already contain a modification of the tryptophan residue at amino acid position 26, with site-directed mutagenesis, a tryptophan modifying chemical reagent or photooxidation to produce a modification or deletion of the tryptophan residue at amino acid position 26, and transforming a desired host cell with a plasmid containing such resulting coding sequence and culturing such host in suitable culture medium and isolating the protein so produced, (ii) substituting or deleting the tryptophan residue at position 26 of the S1 subunit of a Bordetella pertussis toxin amino acid coding sequence, prepared by isolation from native B. pertussis DNA or by synthetic or recombinant DNA techniques, containing an entire or truncated B. pertussis toxin S1 subunit amino acid coding sequence with another amino acid, and transforming a desired host cell with a plasmid containing such resulting coding sequence and culturing such host in suitable culture medium and isolating the protein so produced, or (iii) preparing such coding sequence by synthetic methods and transforming a desired host cell with a plasmid containing such resulting coding sequence and culturing such host in suitable culture medium and isolating the protein so produced. This invention also relates to the protein produced by such process, a vaccine for stimulating protection against whooping cough wherein such vaccine comprises an immunoprotective and non-toxic quantity of such protein, and a method for innoculating a human against subsequent whooping cough which comprises administering the vaccine.

The invention also encompasses a vaccine capable of inducing immunity against pertussis. Such vaccine comprises an immunoprotective and non-toxic amount of the protein of the invention regardless of the method of preparation of such protein. Such vaccine preferably contains 5-25 µg of the protein of this invention, but is not limited to these amounts.

Other antigens which are known to be desirably administered in conjunction with pertussis toxin may also be included in the vaccines. Such additional components are known to those of skill in the art. Preferable additional components include tetanus toxoid and/or diphtheria toxoid as well as filamentous haemagglutinin (FHA) and/or any otner protective antigen of B. pertussis.

The provision of such a vaccine thus allows another aspect of the present invention, i.e., a method for immunizing a human against pertussis which comprises administering the vaccine of the subject invention to such human.

The mode of administration of the vaccine of the invention may be any suitable route which delivers an immunoprotective amount of the protein of the subject invention to the host. However, the vaccine is preferably administered parenterally via the intramuscular or deep subcutaneous routes. Other modes of administration may also be employed, where desired, such as oral administration or via other parenteral routes, i.e., intradermally, intranasally, or intravenously.

The vaccine of the invention may be prepared as a pharmaceutical composition containing an immunoprotective, non-toxic amount of the protein of the invention in a nontoxic and sterile pharmaceutically acceptable carrier. Where the administration of the vaccine is parenteral, the protein of the invention can be optionally admixed or absorbed with any conventional adjuvant, for use as a non-specific irritant to attract or enhance an immune response. Such adjuvants include, among others, aluminum hydroxide, aluminum phosphate, muramyl dipeptide and saponins, such as Quil A. As a further exemplary alternative of the preparation of the vaccine of the invention, the protein of the invention can be encapsulated within microparticles, such as liposomes. In yet another exemplary alternative of the preparation of the vaccine of the invention, the protein of the invention can be administered with an immunostimulating macromolecule, or a tetanus toxoid. Alternatively, an aqueous suspension or solution containing the protein of the invention preferably buffered at physiological pH, may be designed for oral ingestion.

It is preferred that the vaccine of the invention, when in a pharmaceutical preparation, be present in unit dosage forms. The appropriate therapeutically effective dose can be determined readily by those of skill in the art. The effective amount of protein contained in the vaccine of this invention may be in the range of the effective amounts of antigen in conventional B. pertussis acellular or component vaccines, i.e., 5-25 µg of protein. This dose may optionally be delivered with various amounts of filamentous haemagglutinin (FHA) (about 10 ug-25 ug) and/or agglutinogens or other antigens. It will be understood, however, that the specific dose level for any particular patient will depend upon a variety of factors including the age, general health, sex, and diet of the patient; the time of administration; the route of administration; synergistic effects with any other drugs being administered; and the degree of protection being sought. Of course, the administration can be repeated at suitable intervals if necessary. Preferably, three doses will be administered, with 3 to 12 months intervals between each dose, with an optional booster dose later in time.

A further aspect of this invention is a B. pertussis strain in which the chromosomal pertussis toxin gene is inactivated, wherein such strain contains the recombinant DNA molecule of this invention. Another embodiment of the invention is a whole cell vaccine for stimulating protection against whooping cough, wherein such vaccine comprises an immunoprotective and non-toxic amount of the strain of the invention. Such vaccine may comprise such strain alone or in conjunction with other antigens and/or adjuvants. Still another aspect of this invention relates to a method for innoculating a human against subsequent whooping cough which comprises administering the vaccine to such human. Any strain of B. pertussis which contains the chromosomal pertussis gene may be employed as the starting material for preparing the strain of the subject invention. Numerous suitable strains of B. pertussis are publicly available from various repositories such as the American Type Culture Collection, Rockville, Maryland, U.S.A. The preparation of the strain my be carried out by conventional techniques. For example, to prepare the strain of the subject invention, the chromosomal pertussis toxin gene is inactivated by conventional techniques, such as by deletion, point or insertion mutation, and the recombinant DNA molecule of this invention is transformed into such strain by conventional techniques. It is preferable to use a B. pertussis strain in which the chromosomal toxin gene is deleted, rather than inactivated by point or insertion mutations. Inactivation of the gene through point or insertion mutations does not eliminate DNA sequences that have the potential of recombining in vivo with the DNA coding for the mutated pertussis toxin on a autonomously replicating plasmid. On the other hand, in a pertussis toxin gene deletion mutant, no such recombination can occur. The resulting B. pertussis strain is then transformed with the recombinant DNA molecule of this invention to result in the strain of this invention. It is preferable if such recombinant DNA molecule is comprised by the plasmid of this invention. Such recombinant DNA molecule may be integrated into the chromosome of the host cell or, if in the form of a plasmid, may be maintained episomally. After such recombinant DNA molecule are introduced, the resulting B. pertussis strain may be used to produce the mutated pertussis toxin protein of this invention. Alternatively, after such recombinant DNA molecule is introduced, such strain may now be used to prepare a whole cell vaccine against pertussis. Preparation of such a whole cell vaccine is accomplished by conventional techniques.

The provision of such a vaccine thus allows another aspect of the present invention, i.e., a method for immunizing a human against pertussis which comprises administering the vaccine of the subject invention to such human.

The mode of administration of the vaccine of the invention may be any suitable route which delivers an immunoprotective amount of the strain of the subject invention to the host. However, the vaccine is preferably administered parenterally via the intramuscular or deep subcutaneous routes. Other modes of administration may also be employed, where desired, such as oral administration or via other parenteral routes, i.e., intradermally, intranasally, or intravenously.

The appropriate immunoprotective and non-toxic dose of such vaccine can be determined readily by those of skill in the art, i.e., the appropriate immunoprotective and non-toxic amount of the strain of this invention contained in the vaccine of this invention may be in the range of the effective amounts of antigen in conventional B. pertussis whole cell vaccines. This dose may optionally be delivered with various amounts of filamentous haemagglutinin (FHA) (about 10 ug-25 ug) and/or agglutinogens or other antigens. It will be understood, however, that the specific dose level for any particular patient will depend upon a variety of factors including the age, general health, sex, and diet of the patient; the time of administration; the route of administration; synergistic effects with any other drugs being administered; and the degree of protection being sought. Of course, the administration can be repeated at suitable intervals if necessary. Preferably, three doses will be administered, with 3 to 12 months intervals between each dose, with an optional booster dose later in time.

### EXAMPLES

The following examples illustrate the preparation of the coding sequence, vector, transformed host and protein of the invention; antigenic activity of the protein; and preparation of the vaccine of the invention. These examples are illustrative in nature and are not intended to limit the scope of this invention.

In the following Examples, all temperatures are in degrees Centigrade (Celsius). The enzymes used in DNA manipulations were obtained from Boehringer (Mannheim, West Germany) and Amersham (Amersham, England), and were employed according to the supplier's directions.

In the following examples, the following abbreviations may be employed:
YT medium: 8 grams/liter (g/l) Tryptone, 5 g/l NaCl, 5g/l yeast extract
Tris-HCl 1 M stock solution : 121,1 g/l Trizma base + HCl until pH reaches desired value (example 7.5).
LB medium (pH 7.5): Tryptone 10 g/l; yeast extract 5 g/l; NaCl 10 g/l

### Example 1 Construction of expression vector pRIT20001 encoding S1 subunit-related protein

Plasmid pPTX42, described in Locht, C. et al., Nucleic Acids Res., 14:3251 (1986), was digested into fragments with Sau3a. The fragments were resolved by polyacrylamide gel electrophoresis (PAGE) according to the method of Maniatis et al., Molecular Cloning, A Laboratory Manual, Cold Spring Harbor, NY, (1982). A 560 base pair (bp) fragment was purified and inserted into the replicative form of M13mp18, previously digested with BamHI. M13mp18 is described in Messing, J. Methods Enzymol., 101, 20-78, (1983) and was obtained from Bethesda Research Laboratories, Bethesda, Maryland. E. coli TG1 cells, obtained from Amersham, International PLC, Amersham, United Kingdom, were transformed with the replicative form of the recombinant phages according to the technique of Maniatis et al., supra. After transformation and overnight incubation, the phages were analyzed for the presence of the 560 bp insert by DNA sequencing according to the method of Sanger et al., Proc. Natl. Acad. Sci., U.S.A., 74, 5463-5467, (1977).

One phage containing the 560 bp insert was purified and was named pRIT20001. It was determined that the S1 subunit-related protein encoded by pRIT20001 was identical to rS1d, the S1 subunit-related protein encoded by pTXS11 as described in Locht et al., Infect. Immun., 55:2546 (1987), the entire disclosure of which is hereby incorporated herein by reference. In this construct, i.e., pRIT20001, the six amino-terminal amino acids are the first six amino acids from beta-galactosidase. They are followed by five amino acids encoded by the polylinker sequence in the M13mp18 cloning vector, after which residues 2 to 187 of the pertussis toxin S1 subunit coding sequence are present. Finally a Leu residue is encoded by the polylinker and a Stop codon is provided.

### Example 2 Synthesis of S1 related protein in E. coli using pRIT20001

1.5 ml of an overnight culture of E. coli TG1 cells were inoculated in 100 ml YT medium, and were thereafter infected with 10 µl of pRIT20001, prepared as described in Example 1, or M13mp18 phage stock. The infected cells were grown under constant shaking at 37° until the Optical Density (OD) reached 0.3. Isopropyl-beta-D-galactopyranoside (IPTG) was then added to the cells to a final concentration of 1 mM, and growth was continued for 4 to 5 hours. The cells were then harvested by centrifugation, resuspended in 3 ml lysis buffer (25 mM Tris-HCl (pH 7.5), 25 mM NaCl) and lysed by two passages through a French pressure cell. The lysate was fractionated by centrifugation at 12,000 x G for 40 minutes.

The supernatant fraction was then analyzed for the presence of the S1 related protein by Western blot according to the method of Towbin et al., Proc. Natl. Acad-Sci., U.S.A., 76:4350-4354, (1979), and detected with an anti-S1 monoclonal antibody B2F8 [See, Marchitto, K.S. et al., Infect. Immun. 55:1309 (1987)], provided by Dr. J.M. Keith, Laboratory of Pathobiology, NIAID, Rocky Mountain Laboratories, Hamilton, Montana. Supernatant fractions from cells infected with M13mp18 did not contain a protein specifically recognized by monoclonal antibody B2F8. However, supernatant fractions from cells infected with pRIT20001 contained a protein of about 22,000 daltons that reacted with monoclonal antibody B2F8 on the Western blot. This protein behaved the same way with monoclonal antibody B2F8 on the Western blot as the S1 subunit-related protein, rS1d, encoded by pTXS11 [see, Locht, C. et al., Infect. Immun., (1987), supra.]

### Example 3. Enzymatic activities of S1 subunit-related protein synthesized in E. coli infected by pRIT20001

The supernatant fraction described in Example 2 were assayed for the presence of S1 subunit-related protein specific enzymatic activities. The level of these activities were compared to the activities of rS1d which had been partially purified from E. coli cells containing PTXS11. In the nicotinamide adenine dinucleotide (NAD)-glycohydrolase assay-described in Locht et al., Infect. Immun., (1987), supra, the release of nicotinamide from NAD catalyzed by such partially purified rS1d was measured. 1 µg of such partially purified rS1d catalyzed the release of 1,200 pmoles of nicotinamide after three hours of reaction at 30° in the assay conditions described in Locht et al., Infect. Immun., (1987), supra. The supernatant fraction of TG1 cells infected with M13mp18 contained no significant NAD-glycohydrolase activity, and the measured nicotinamide release values were not different from the background levels. 25 µl of the supernatant fraction of E. coli TG1 cells infected with pRIT20001, however, catalyzed the release of 300 pmoles nicotinamide from NAD after three hours of incubation in the same conditions. The substantial reduction of the enzymatic activity by the supernatant of pRIT20001 infected E. coli cells is due to the presence of an inhibitor in the crude E. coli cell extracts (C. Locht, unpublished observation).

The ability of the partially purified rSld and the supernatant fractions of pRIT20001 infected E. coli cells and M13mp18 infected TG1 cells to catalyze the S1-specific adenosine diphosphate (ADP)-ribosylation of the 41,000 dalton signal transducing GTP-binding protein Gi in CHO cell membranes was also investigated. This assay is also described in Locht et al., Infect. Immun., (1987), supra. When the partially purified rS1d or pertussis toxin was incubated in the presence of radiolabeled NAD and Chinese Hamster Ovary (CHO) cell membranes in the conditions described in Locht et al., and the products of the reaction were analyzed by sodium dodecyl sulfate-PAGE (SDS-PAGE) and autoradiography, specific labeling of the 41,000 dalton Gi protein was detected. No labeling of the Gi protein was seen when rSld or pertussis toxin was replaced by the lysis buffer (25 mM Tris-HCl (pH7.5), 25 mM NaCl) or the supernatant fraction of E. coli cells infected with M13mp18. In contrast, the supernatant fraction of cells infected with pRIT20001 (containing the protein encoded by a truncated S1 subunit coding sequence) was able to catalyze the incorporation of radiolabeled ADP-ribose in the 41,000 dalton Gi protein.

### Example 4 Chemical modification of residue Trp-26 in the S1 subunit-related protein encoded by pRIT20001

The enzymatically active S1 related protein encoded by pRIT20001, and prepared according to Example 2, contains only a single Trp residue located at position 26 of the mature S1 protein, i.e., 15 residues upstream from the cysteine residue at position 41.

The S1 subunit-related protein prepared according to Example 2 was partially purified and incubated with a 1000-fold molar excess of 2-hydroxy-5-nitrobenzyl bromide (HNBB) dissolved in dry acetone. HNBB modifies tryptophan residues by derivatization. The reaction was performed in a 0.1 M sodium acetate buffer (pH 4.0), containing 2M urea at 25° for 2 hours. The solution was then dialyzed against 0.1 M sodium phosphate (pH 4.0) for 15 hours at 4°. In parallel, the S1 subunit-related protein prepared according to Example 2 was treated the same way, except that HNBB was omitted in the reaction mixture.

The S1 subunit-related protein prepared according to Example 2 as well as purified HNBB treated and HNBB untreated S1 subunit-related protein prepared as described above, hereinafter referred to as "untreated S1 subunit related protein" "HNBB treated S1 subunit-related protein" and "HNBB untreated S1 subunit-related protein", respectively, were then analyzed for their ability to catalyze NAD-glycohydrolysis and ADP-ribosyltransfer to Gi in CHO cell membranes using assay conditions as described in Locht et al., Infect. Immun., (1987), supra. Untreated S1 subunit-related protein as well as HNBB untreated S1 subunit-related protein expressed NAD-glycohydrolase activity in a concentration-related fashion. Only marginal NAD-glycohydrolase activity could be detected when HNBB treated S1 subunit-related protein was assayed, and the activity was reduced to about 5% when a smaller concentration, i.e., 1 µg, of the HNBB treated S1 subunit-related protein was assayed. When 0.5 µg and 1.0 µg of untreated, HNBB treated and HNBB untreated S1 subunit-related protein were assayed for their ADP-ribosyltransferase activity on the Gi protein in CHO cell membranes, only the untreated S1 subunit-related protein and the HNBB untreated S1 subunit related protein were found to express Gi-specific ADP-ribosylation activity at both concentrations. No Gi:ADP-ribosyltransferase activity could be detected at either concentration of HNBB treated S1 subunit-related protein.

### Example 5 Construction of a coding sequence of the invention using site-directed mutagenesis

Single stranded DNA of phage pRIT20001, prepared according to Example 1, was purified and submitted to oligonucleotide-driven site-directed mutagenesis using the "Oligonucleotide-Directed in vitro Mutagenesis System" commercialized by Amersham International PLC, Amersham, United Kingdom, code RPN2322, under the conditions recommended by the supplier. The oligonucleotide used to specifically delete the codon TGG coding for the tryptophan residue at the 26th amino acid position of the rSld gene in pRIT20001 had the sequence 5' CGTTGTTTCCCGCCGTGAAT 3'. The oligonucleotide used to specifically change the codon TGG coding for the tryptophan residue at the 26th amino acid position of the S1 gene in pRIT20001 had the sequence

5' CGTTGTTTCCGGTCGCCGTGAAT 3'

The mutant candidates were screened by differential hybridization with the mutagenic oligonucleotides by the technique of Wallace, in Suggs, Developmental Biology Using Purified Genes, Ed. D. Brown, N.Y., Academic Press. The mutations were then verified by DNA sequencing by the technique of Sanger et al., (1977) supra. One candidate, named pRIT20002 and containing the Trp-2E deletion mutation, was cloned and further analyzed for its ability to express the Trp-26 modified S1 gene as described in Example 6, infra. One candidate, named pRIT20008 and containing the Trp-26 to Thr substitution mutation was cloned and further analyzed for its ability to express the Trp-26 modified S1-related gene as described in Example 6, infra.

### Example 6 Expression of a Trp-26 modified rS1d genes in E. coli and analysis of the enzymatic activities of their products

E. coli TG1 cells were infected with pRIT20002 or pRIT20008, both prepared as described in Example 5, and expression of the Trp-26 modified S1 subunit-related genes containing the deletion of the Trp-26 codon TGG or its substitution by codon ACC for Threonine was induced with IPTG as described in Example 2. Cells were then harvested and lysed, the cell lysates fractionated by centrifugation, and the supernatant fractions analyzed for the presence of the mutated rS1d by Western blot and reaction with the anti-S1 monoclonal antibody B2F8, all of the above methods carried out as described in Example 2.

The results were compared to the results obtained in Example 2. The supernatant fractions of cells infected by pRIT20001, pRIT20002 or pRIT20008 contained a protein that reacted with monoclonal antibody B2F8 on Western blots, whereas supernatant fractions of cells infected with M13mp18 did not contain such a protein. Furthermore, the B2F8-reactive proteins in the pRIT20001, pRIT20002 and pRIT20008 supernatants showed the same apparent molecular weight as the partially purified rS1d referred to in Example 3. Based on visual inspection of the Western blots, pRIT20001, pRIT20002 and pRIT20008 expressed roughly similar amounts of their respective S1 subunit-related proteins.

The pRIT20001, pRIT20002 and pRIT20008 supernatant fractions were compared for their level of NAD-glycohydrolase and ADP-ribosyltransferase activities in the assay systems described in Example 3. Whereas 25 µl of the pRIT20001 supernatant fraction again catalyzed the release of-300 pmoles nicotinamide in a three hour reaction, the pRIT20002 and pRIT20008 supernatant fractions showed NAD-glycohydrolase activity levels similar to supernatant fractions from cells infected with M13mp18 i.e., substantially no NAD-glycohydrolase activity. Similarly, although the pRIT20001 supernatant fraction was able to catalyze the ADP-ribosylation of the Gi protein, no such activity could be detected with the pRIT20002 supernatant fraction or with the pRIT20008 supernatant fraction. Thus, the S1 subunit related proteins encoded by pRIT20002 and pRIT20008 had substantially inactivated S1 subunit protein enzymatic activities.

### Example 7 Antigenic structure of the S1 subunit-related protein encoded by pRIT20002 and pRIT20008

The pRIT20002 and pRIT20008 supernatant fractions, prepared as described in Example 6, were analyzed for their ability to react with monoclonal as well as polyclonal anti-pertussis toxin antisera by Western blots. Polyclonal antibodies to pertussis toxin, obtained from E. Simoen, SmithKline-R.I.T., s.a., Rixensart, Belgium, (SK-RIT), as well as several monoclonal anti-pertussis toxin antibodies,obtained from M. Francotte (SK-RIT), and J.G. Kenimer (Laboratory of Cellular Physiology, Food and Drug Administration, Bethesda, MD), were found to react specifically with the pRIT20002 and pRIT20008 derived S1 subunit related proteins in the crude E. coli cell supernatants. Some of the reacting monoclonal antibodies had good toxin neutralizing and protective activities in mouse challenge systems. This result indicates that at least some protective epitopes are still conserved in the pRIT20002 and pRIT20008 derived proteins, and that the protein has utility in a vaccine against whooping cough.

### Example 8 Reconstruction of the complete pertussis toxin operon containing a Trp-26 deletion in the S1 subunit cistron

A 4.7 kilo base pair (kbp) DNA fragment, obtained from T. Cabezon (SK-RIT), containing the complete pertussis toxin operon was inserted into pUC7 which had been previously digested with EcoRI. Such insertion was accomplished according to the method of Maniatis et al. supra. PUC7 is described by J. Vieira & J. Messing, Gene 19:259-268 (1982).

The resulting recombinant plasmid was introduced into E. coli TG1 competent cells according to the method of Maniatis et al., supra. The transformed cells were cloned by the method of Maniatis et al., supra and the clones analyzed by restriction digests of their plasmids by the method of Maniatis et al., supra. One plasmid, named pRIT13070 and containing the entire pertussis toxin operon, was purified and then digested with AccI according to the method of Maniatis et al., supra. The resulting two fragments were separated by polyacrylamide gel electrophoresis (PAGE) and the larger one, was purified and used in the next ligation step.

The replicative form of pRIT20002 was purified and also digested with AccI. The resulting fragments were separated by PAGE and the 300 bp DNA fragment containing the Trp-26 deletion was purified. The 300 bp AccI fragment from pRIT20002 was then ligated into the large AccI fragment from pRIT13070, according to the method of Maniatis et al., supra, and the resulting recombinant plasmid was introduced into E. coli TG1 competent cells by the method of Maniatis et al., supra. After cloning, plasmid pRIT13071 containing the entire pertussis toxin operon was isolated by the method of Maniatis et al., supra. pRIT13071 contains the deletion of the Trp-26 codon in the S1 cistron, but is otherwise identical to pRIT13070.

The Trp26 modified pertussis toxin operon contained by pRIT13070 can now be used to introduce the modification into the B. pertussis genome by exchange of the resident wild type operon with the modified operon by homologous recombination. Alternatively, the modified operon can also be incorporated in a plasmid which is subsequently transformed into a Bordetella cell or any other appropriate host cell for expression of the protein encoded by the Trp-26 modified operon.

Such modified Bordetella strains will then be used to the coding sequence of this invention and/or other protective antigens for example FHA and/or agglutinogens. These antigens will then be used as vaccines against whooping cough with appropriate adjuvants such as aluminum adjuvants.

### Example 9 Parenteral Vaccine preparation

5-25 µg of the protein encoded by pRIT20002 or pRIT20008 (Example 6) is mixed with an aluminum adjuvant such as aluminum hydroxide, to produce a vaccine in a form appropriate for incorporation into a parenteral administration dosage form.

It is appreciated that the invention is not limited to the particular embodiments described above in the Examples. All embodiments of the invention, therefore, are believed to come within the scope of the following claims.

### Example 10. Expression of the mutated pertussis toxin gene in Bordetella pertussis

Plasmid pRIT13071, prepared according to the method of Example 8, was digested with restriction enzyme EcoRI, and the entire pertussis toxin operon containing the deletion of the Trp-26 codon in the S1 cistron was isolated as a 4.7 kilobase pair (kbp) DNA fragment by the methods of Maniatis et al., supra. This 4.7 kbp DNA fragment was then inserted into the single EcoRI site of the low copy number plasmid pLAFRII to result in plasmid pRIT13268. Plasmid pLAFRII is equivalent to pLAFRI described by Friedman et al., Gene, 18:289-296 (1982), and was provided by Dr. J.J. Mekalanos, Harvard Medical School, Cambridge, Massachusetts, U.S.A. The recombinant plasmids were transformed into E. coli strain SM10 which was, provided by Dr. S. Stibitz, U.S. Food and Drug Administration, Bethesda, Maryland, U.S.A. pLAFRII carries a tetracyclin resistance gene and therefore SM10 transformants were selected on LB medium (Maniatis et al., supra.) containing 12.5 µg/ml tetracyclin (Maniatis et al., supra.). The tetracyclin resistant transformants were further analyzed by DNA sequencing (Sanger et al., Proc. Natl. Acad. Sci. USA, (1977), supra) for the presence of the mutated pertussis toxin gene. The tetracyclin resistant SM10 clones containing the modified pertussis toxin gene were then conjugated with Bordetella pertussis strain SK39. Strain SK39 is described by Knapp, and Mekalanos, J. Bacteriol., 170, 5059-5066 (1988) and was provided by Dr. J.J. Mekalanos, supra. The SK39 strain contains a Tn5 insertion in the S1 cistron of the pertussis toxin operon in the chromosome. Because of this interruption of the toxin gene, this strain does not synthesize pertussis toxin. Tetracyclin resistant B. pertussis SK39 exconjugates were identified on BC agar (Difco Laboratories, Detroit, Michigan, U.S.A.) supplemented with 250 ml of defibrinated sheep blood per liter (BG medium) and 20 µg/ml tetracyclin. The tetracyclin resistant clones were then grown in the modified Stainer-Scholte broth described by Kloos, et al. in C.R. Manclark and J.C. Hill (eds.), International Symposium on Pertussis, U.S. Department of Health, Education, and Welfare, Washington, D.C., pp. 70-80 (1979). After growth, the cells were separated from the culture medium by centrifugation. Both the cells and the culture medium were then analyzed by Western Blot as described by Locht et al., Infect. Immun. (1987), supra, for the presence of pertussis toxin either as a cell associated form or free in the culture medium. The results indicate that B. pertussis strain SK39 containing the recombinant plasmid does synthesize pertussis toxin and that the toxin is released in the culture supernatant, although significant amounts of pertussis toxin can be found in a cell-associated form. B. pertussis strain SK39 without the recombinant plasmid did not contain detectable amounts of pertussis toxin in either the culture medium or associated to the bacterial cells. It should also be noted that the B. pertussis strain SK39 containing the recombinant plasmid is useful in a whole cell vaccine. Such vaccine is prepared according to conventional techniques.

Using the methods of Examples 8 and 10, but using pRIT20008 instead of pRIT20002, plasmid pRIT13269 was created. pRIT13269 contains the complete pertussis toxin coding sequence but threonine (Thr) is substituted for the tryptophan mutation at amino acid position 26 in the S1 coding sequence.

### Example 11 Biological activity of the mutated pertussis toxin

Purified pertussis toxin, purchased from List Biologicals, Campbell, California, U.S.A., as well as culture supernatants from toxin synthesizing B. pertussis strain Tohama I, induce morphological changes in Chinese Hamster Ovary (CHO) cells, as described by Hewlett et al., Infect. Immun., 40:1198-1203 (1983). On the other hand, the culture supernatant of B. pertussis strain SK39 containing the recombinant plasmid with the modified pertussis toxin gene induced no detectable morphological changes in the CHO cells, indicating that the deletion of the Trp-26 codon in the S1 subunit cistron of the pertussis toxin gene drastically reduced the biological activity of pertussis toxin. When the activity of the mutant toxin was-compared on a more quantitative basis, the mutant appeared at least 500 fold less toxic in this CHO cell assay.

### Example 12 Construction of a B. pertussis strain in which the pertussis toxin operon is deleted

To express an entire or truncated B. pertussis toxin S1 subunit amino acid coding sequence which contains a modification of the tryptophan residue at amino acid position 26, it is preferable to use a B. pertussis recipient strain in which the chromosomal toxin gene is deleted, rather than inactivated by point or insertion mutations. Inactivation of the gene through point or insertion mutations does not eliminate DNA sequences that have the potential of recombining in vivo with the DNA coding for the mutated pertussis toxin on a autonomously replicating plasmid. On the other hand, in a pertussis toxin gene deletion mutant, no such recombination can occur. Therefore, the pertussis toxin coding sequences of B. pertussis Tohama I strain, were deleted in the following way. First, the B. pertussis Tohama I strain was plated on BG medium (supra) containing 400 µg/ml streptomycin. Streptomycin resistant strains were then plated on BG medium (supra) containing 50 µg/ml nalidixic acid. The B. pertussis Tohama I streptomycin and nalidixic resistant strains were used for the conjugation experiments.
Plasmid pTOX9, described by Black and Falkow, Infect. Immun., 55:2465-2470 (1987), and provided by Dr. W.J. Black, Stanford University Medical School, Stanford, California, U.S.A., contains the pertussis toxin gene on an approximately 10 kbp B. pertussis DNA fragment. pTOX9 was digested with KpnI and BglII restriction enzymes and then with Bal31 exonuclease. After ligation, the deleted pertussis toxin gene was isolated on an approximately 7 kbp Clal/Clal DNA fragment from the Bal31 treated pTOX9. All the methods used for the treatment of pTOX9 are described by Maniatis et al. (1982), supra. The 7 kbp DNA fragment was then inserted into the unique HindIII site of plasmid pSORTP1 a derivative of pRTPI containing a gentamycin-resistant gene, described by Stibitz, et al., Gene, 50:133-140 (1986), which was provided by Dr. S. Stibitz, supra. The recombinant plasmid was transformed into E. coli strain SM10 (supra) and analyzed by DNA sequencing to identify the deletion mutation. E. coli SM10 containing the recombinant plasmid with the desired deletion was then conjugated to the streptomycin, nalidixic acid resistant B. pertussis strain Tohama I. The exconjugates containing the recombinant plasmid inserted into the chromosome were identified by their resistance to gentamicin and their sensitivity to streptomycin on BG agar plates (supra). Plasmid pSORTP1 contains the gene for resistance to gentamicin and the gene for the dominant sensitivity to streptomycin, and is not able to autonomously replicate in B. pertussis. Therefore, gentamicin resistant,
streptomycin sensitive B. pertussis strains can only be generated if the recombinant plasmid is inserted in the chromosome by homologous recombination. Such homologous recombination possibly involves the toxin gene flanking regions in the chromosome and the recombinant plasmid. The gentamicin resistant clones were then plated onto BG plates containing 400 µg/ml streptomycin (supra) to select for streptomycin resistant B. pertussis revertants. Some of these revertants appeared because of a second homologous recombination between the chromosome and the inserted plasmid. The resulting streptomycin resistant strains were analyzed for the loss of gentamicin resistance. These clones were then analyzed by Southern blot hybridization, as described by Maniatis et al. (1982), supra, for the loss of the pertussis toxin gene in the chromosome. The resulting B. pertussis strain (hereinafter referred to as "BPRA") is then used for the introduction of plasmids containing the mutated pertussis toxin genes, such as the pLAFRII-derived plasmids described in Example 10 such as pRIT13268. After such plasmids are introduced, the resulting B. pertussis strain BPRA(pRIT13268) is used to produce mutated pertussis toxin. Alternatively, after such plasmids are introduced, the resulting B. pertussis strain is useful in a whole cell vaccine. Such whole cell vaccine is prepared according to conventional techniques well known in the art.

### Example 13. Expression in Bordetella pertussis of pertussis toxin genes containing single or double mutations

BPRA, the Bordetella pertussis strain in which the pertussis toxin structural gene was deleted, prepared as described in Example 12, was used as a recipient strain for plasmids derived from pLAFRII. Plasmid pLAFRII is described in Example 10. The pLAFRII-derived plasmids contained the pertussis toxin gene with either single or double mutations. The single mutants contained either a deletion or substitution of Trp-26. The genetic manipulations were carried out using classical subcloning techniques as described by Maniatis et al. (1982), supra. Site-directed mutagenesis was performed using the "Oligonucleotide-directed in vitro mutagenesis system" commercialized by Amersham International plc., Amersham United Kingdom, code RPN2322, as described for the Trp-26 mutation exemplified in Example 5. After mutagenesis, all mutants were completely sequenced using the-DNA sequencing technique described by Sanger et al., Proc. Natl. Acad. Sci. USA (1977), supra. The pLAFRII-derived plasmids containing the pertussis toxin gene with the different mutations were transformed into E. coli strain SM10, and then were conjugated into the recipient B. pertussis strain by the procedure described in Example 10, with the exception that the B. pertussis recipient strain in this case is BPRA, the strain in which the chromosomal pertussis toxin structural gene has been deleted as described in Example 12. The B. pertussis exconjugants were analyzed by Western blot, as described in Example 10, for the presence of pertussis toxin.

The mutant toxins were then compared to purified pertussis toxin (purchased from List Biological Laboratories, Campbell, California), to the wild type pertussis toxin in the supernatant fraction of B. pertussis Tohama I (supra), and to the supernatant fraction of the strain of B. pertussis in which the chromosomal toxin gene was deleted. Such comparison was based on the biological activities on CHO cells as described in Example 11. It was observed that 3 ng of purified pertussis toxin caused a significant cytotoxic clustering of the CHO cells. These morphological changes were also observed when the culture supernatant of B. pertussis Tohama I was assayed. In contrast, all the mutant toxins in the form of culture supernatant induced only background CHO cell cytotoxicity. On a quantitative basis, all the mutant toxins were estimated to be at least 500 fold less toxic than the wild type pertussis toxin.

The above description fully discloses the invention including preferred embodiments thereof. Modifications and improvements of the embodiments specifically disclosed herein are within the scope of the following claims.

## Claims

1. A Bordetella pertussis toxin coding sequence containing an entire or truncated B. pertussis toxin S1 subunit amino acid coding sequence provided that (a) the S1 subunit coding sequence contains a modification of the tryptophan residue at amino acid position 26 (hereinafter "Trp-26"), and (b) the Trp-26 modification results in substantially inactivated S1 enzymatic activity in the protein encoded by the B. pertussis toxin coding sequence but such protein has retained the capacity to be recognized by anti-pertussis toxin antibodies.

2. The B. pertussis toxin coding sequence of Claim 1 wherein said coding sequence comprises the entire Bordetella pertussis toxin operon amino acid coding sequence.

3. The B. pertussis toxin coding sequence of Claim 1 wherein said coding sequence comprises a truncated Bordetella pertussis toxin operon.

4. The B. pertussis toxin coding sequence of Claim 1 wherein said coding sequence comprises only the S1 subunit amino acid coding sequence.

5. The B. pertussis toxin coding sequence of Claim 1 wherein said coding sequence comprises only a truncated portion of the S1 subunit amino acid coding sequence.

6. The B. pertussis toxin coding sequence of Claim 5 wherein said coding sequence comprises only amino acids 2 through 187 of the S1 subunit amino acid coding sequence.

7. The B. pertussis toxin coding sequence of Claim 1 wherein said coding sequence additionally comprises at least one other coding sequence.

8. The B. pertussis toxin coding sequence of Claim 7 which has the following coding sequence:

ATG ACC ATG ATT ACG AAT TCG AGC TCG GTA CCC GGG GAT
(Met) Thr Met Ile Thr Asn Ser Ser Ser Val Pro Gly Asp
beta-galactosidase polylinker 2
S1

CCT CCC GCC ACC GTA......GTA GCG TCG ATC CTC TAG
Pro Pro Ala Thr Val........Val Ala Ser Ile Leu Stop
3 4 5 6 7 184 185 186 187 polylinker
S1

9. The B. pertussis toxin coding sequence of Claim 1 wherein said coding sequence additionally comprises a modification of an additional amino acid residue in the S1 subunit coding sequence, provided that the protein encoded by the B. pertussis toxin coding sequence retains the capacity to be recognized by anti-pertussis toxin antibodies, that the additional modification does not include deletion or substitution of the glutamic acid residue at position 129, and that when the tryptophan residue at position 26 of the S1 subunit coding sequence is substituted by isoleucine, the additional modification does not include substitution of the aspartic acid residue at amino acid position 11 by serine, substitutioin of the arginine residue at amino acid position 9 by lysine or glycine, substitution of the phenylalanine residue at position 50 by glutamic acid, substitution of the arginine residue at position 13 by leucine, substitution of the aspartic acid residue at position 1 by glutamic acid, substitution of the glutamic acid residue at position 39 by glycine, substitution of the tyrosine residue at position 130 by glycine, substitution of the glycine residue at position 86 by glutamic acid, substitution of the isoleucine residue at position 88 by glutamic acid, substitution of the tyrosine residue at position 89 by serine, substitution of the tyrosine residue at position 8 by aspartic acid or substitution of the threonine residue at position 53 by isoleucine.

10. The B. pertussis toxin coding sequence of Claim 9 wherein the additional modification occurs at the histidine residue at amino acid position 35 of the S1 subunit coding sequence (His-35); the arginine residue at amino acid position 9 of the S1 subunit coding sequence (Arg-9) or the serine residue at amino acid position 40 of the S1 subunit coding sequence (Ser-40).

11. A recombinant DNA molecule comprising the Bordetella pertussis toxin coding sequence of Claim 1, 2, 3, 4, 5, 6, 7, 8, 9 or 10.

12. A recombinant DNA plasmid comprising the recombinant DNA molecule of Claim 12 in operative association with suitable expression control sequences.

13. The recombinant plasmid of Claim 12 in which the modification of the tryptophan residue at position 26 of the S1 subunit coding sequence is a deletion mutation.

14. The recombinant plasmid of Claim 12 in which the modification of the tryptophan residue at position 26 of the S1 subunit coding sequence is a substitution mutation.

15. The recombinant plasmid of Claim 14 in which the tryptophan residue at position 26 is substituted by threonine.

16. A host cell transformed with the plasmid of Claim 12.

17. The host cell of Claim 16 which is Bordetella pertussis strain in which the pertussis toxin operon is deleted.

18. Protein encoded by the coding sequence of Claim 1, 2, 3, 4, 5, 6, 7, 8, 9 or 10.

19. A vaccine for stimulating protection against whooping cough wherein such vaccine comprises an immunoprotective and non-toxic quantity of the protein encoded by the recombinant DNA molecule of Claim 1.

20. A process for producing a transformed host cell which comprises transforming a desired host cell with the recombinant DNA plasmid containing the coding sequence of Claim 1.

21. A process for producing the protein encoded by a Bordetella pertussis toxin amino acid coding sequence containing an entire or truncated B. pertussis toxin S1 subunit amino acid coding sequence provided that (a) the S1 subunit coding sequence contains a modification of the tryptophan residue at amino acid position 26 (hereinafter "Trp-26"), and (b) the Trp-26 modification results in substantially inactivated S1 enzymatic activity in the protein encoded by the B. pertussis toxin operon amino acid coding sequence but such protein has retained the capacity to be recognized by anti-pertussis toxin antibodies, wherein such process comprises culturing a host cell transformed with a recombinant DNA plasmid comprising the recombinant DNA coding sequence of Claim 1 in suitable culture medium and isolating the protein so produced.

22. A process for producing the protein encoded by a Bordetella pertussis toxin amino acid coding sequence containing an entire or truncated B. pertussis toxin S1 subunit amino acid coding sequence provided that (a) the S1 subunit coding sequence contains a modification of the tryptophan residue at amino acid position 26 (hereinafter "Trp-26"), and (b) the Trp-26 modification results in substantially inactivated S1 enzymatic activity in the protein encoded by the B. pertussis toxin operon amino acid coding sequence but such protein has retained the capacity to be recognized by anti-pertussis toxin antibodies, wherein such process comprises (i) treating a Bordetella pertussis toxin amino acid coding sequence, prepared by isolation from native B pertussis DNA or by synthetic or recombinant DNA techniques, containing an entire or truncated B. pertussis toxin S1 subunit amino acid coding sequence, provided that the S1 subunit coding sequence does not already contain a modification of the tryptophan residue at amino acid position 26, with site-directed mutagenesis, a tryptophan modifying chemical reagent or photooxidation to produce a modification or deletion of the tryptophan residue at amino acid position 26, and transforming a desired host cell with a plasmid containing such resulting coding sequence and culturing such host in suitable culture medium and isolating the protein so produced, (ii) substituting or deleting the tryptophan residue at position 26 of the S1 subunit of a Bordetella pertussis toxin amino acid coding sequence, prepared by isolation from native B. pertussis DNA or by synthetic or recombinant DNA techniques, containing an entire or truncated B. pertussis toxin S1 subunit amino acid coding sequence with another amino acid, and transforming a desired host cell with a plasmid containing such resulting coding sequence and culturing such host in suitable culture medium and isolating the protein so produced, or (iii) preparing such coding sequence by synthetic methods and transforming a desired host cell with a plasmid containing such resulting coding sequence and culturing such host in suitable culture medium and isolating the protein so produced.

23. A B. pertussis strain which contains an inactivated chromosomal pertussis toxin gene which additionally comprises the coding sequence of Claim 1.

24. A vaccine for stimulating protection against whooping cough wherein such vaccine comprises an immunoprotective and non-toxic quantity of the strain of Claim 23.

## Patentansprüche

1. Bordetella pertussis-Toxin codierende Sequenz, enthaltend eine vollständige oder verkürzte, die Aminosäuresequenz der S1-Untereinheit des B. pertussis-Toxins codierende Sequenz, mit der Maßgabe, daß (a) die die S1-Untereinheit codierende Sequenz eine Modifikation des Tryptophanrestes an der Aminosäureposition 26 (nachstehend "Trp-26") enthält, und (b) die Trp-26-Modifikation eine im wesentlichen inaktivierte S1-Enzymaktivität bei dem Protein zur Folge hat, das von der das B. pertussis-Toxin codierenden Sequenz codiert wird, wobei aber das Protein die Fähigkeit behalten hat, von Antikörpern gegen das Pertussis-Toxin erkannt zu werden.

2. B. pertussis-Toxin codierende Sequenz nach Anspruch 1, wobei die codierende Sequenz die vollständige, die Aminosäuresequenz des Bordetella pertussis-Toxin-Operons codierende Sequenz umfaßt.

3. B. pertussis-Toxin codierende Sequenz nach Anspruch 1, wobei die codierende Sequenz ein verkürztes Bordetella pertussis-Toxin-Operon umfaßt.

4. B. pertussis-Toxin codierende Sequenz nach Anspruch 1, wobei die codierende Sequenz nur die die Aminosäuresequenz der S1-Untereinheit codierende Sequenz umfaßt.

5. B. pertussis-Toxin codierende Sequenz nach Anspruch 1, wobei die codierende Sequenz nur die die Aminosäuresequenz des verkürzten Anteils der S1-Untereinheit codierende Sequenz umfaßt.

6. B. pertussis-Toxin codierende Sequenz nach Anspruch 5, wobei die codierende Sequenz nur die die Aminosäuresequenz der Aminosäuren 2 bis 187 der S1-Untereinheit codierende Sequenz umfaßt.

7. B. pertussis-Toxin codierende Sequenz nach Anspruch 1, wobei die Sequenz zusätzlich mindestens eine weitere codierende Sequenz umfaßt.

8. B. pertussis-Toxin codierende Sequenz nach Anspruch 7 mit der folgenden codierenden Sequenz:

ATG ACC ATG ATT ACG AAT TCG AGC TCG GTA CCC GGG GAT
(Met) Thr Met Ile Thr Asn Ser Ser Ser Val Pro Gly Asp
ß-Galactosidase Polylinker 2
S1

CCT CCC GCC ACC GTA......GTA GCG TCG ATC CTC TAG
Pro Pro Ala Thr Val........Val Ala Ser Ile Leu Stop
3 4 5 6 7 184 185 186 187 Polylinker
S1

9. B. pertussis-Toxin codierende Sequenz nach Anspruch 1, wobei die codierende Sequenz zusätzlich eine Modifikation eines weiteren Aminosäurerestes in der die S1-Untereinheit codierenden Sequenz umfaßt, mit der Maßgabe, daß das Protein, das von der das B. pertussis-Toxin codierenden Sequenz codiert wird, die Fähigkeit behält, von Antikörpern gegen das Pertussis-Toxin erkannt zu werden, daß die zusätzliche Modifikation eine Deletion oder Substitution des Glutaminsäurerestes an der Position 129 nicht einschließt, und daß im Falle der Substitution des Tryptophanrestes an der Position 26 der die S1-Untereinheit codierenden Sequenz durch Isoleucin, die zusätzliche Modifikation keine Substitution des Asparaginsäurerestes an der Aminosäureposition 11 durch Serin einschließt, keine Substitution des Argininrestes an der Aminosäureposition 9 durch Lysin oder Glycin, keine Substitution des Phenylalaninrestes an der Position 50 durch Glutaminsäure, keine Substitution des Argininrestes an der Position 13 durch Leucin, keine Substitution des Asparaginsäurerestes an der Position 1 durch Glutaminsäure, keine Substitution des Glutaminsäurerestes an der Position 39 durch Glycin, keine Substitution des Tyrosinrestes an der Position 130 durch Glycin, keine Substitution des Glycinrestes an der Position 86 durch Glutaminsäure, keine Substitution des Isoleucinrestes an der Position 88 durch Glutaminsäure, keine Substitution des Tyrosinrestes an der Position 89 durch Serin, keine Subtitution des Tyrosinrestes an der Position 8 durch Asparaginsäure oder keine Substitution des Threoninrestes an der Position 53 durch Isoleucin.

10. B. pertussis-Toxin codierende Sequenz nach Anspruch 9, wobei die zusätzliche Modifikation am Histidinrest an der Aminosäureposition 35 der die S1-Untereinheit codierenden Sequenz (His-35) stattfindet; am Argininrest an der Aminosäureposition 9 der die S1-Untereinheit codierenden Sequenz (Arg-9) oder am Serinrest an der Aminosäureposition 40 der die S1-Untereinheit codierenden Sequenz (Ser-40).

11. Rekombinantes DNA-Molekül, das die das Bordetella pertussis-Toxin codierende Sequenz nach Anspruch 1, 2, 3, 4, 5, 6, 7, 8, 9 oder 10 umfaßt.

12. Rekombinantes DNA-Plasmid, das das rekombinante DNA-Molekül nach Anspruch 11 in funktioneller Verbindung mit geeigneten Expressions-Kontrollsequenzen umfaßt.

13. Rekombinantes Plasmid nach Anspruch 12, bei dem die Modifikation des Tryptophanrestes an Position 26 der die S1-Untereinheit codierenden Sequenz eine Deletionsmutation ist.

14. Rekombinantes Plasmid nach Anspruch 12, bei dem die Modifikation des Tryptophanrestes an der Position 26 der die S1-Untereinheit codierenden Sequenz eine Substitutionsmutation ist.

15. Rekombinantes Plasmid nach Anspruch 14, bei dem der Tryptophanrest an der Position 26 durch Threonin substituiert ist.

16. Wirtszelle, die mit dem Plasmid nach Anspruch 12 transformiert ist.

17. Wirtszelle nach Anspruch 16, die ein Bordetella pertussis-Stamm ist, bei dem das Pertussis-Toxin-Operon deletiert ist.

18. Protein, das von der codierenden Sequenz nach Anspruch 1, 2, 3, 4, 5, 6, 7, 8, 9 oder 10 codiert wird.

19. Impfstoff zur Stimulierung von Schutz gegen Keuchhusten, der eine immunprotektive und nicht-toxische Menge des Proteins umfaßt, das von dem rekombinanten DNA-Molekül nach Anspruch 1 codiert wird.

20. Verfahren zur Herstellung einer transformierten Wirtszelle, das die Transformation einer gewünschten Wirtszelle mit dem rekombinanten, die codierende Sequenz nach Anspruch 1 enthaltenden DNA-Plasmid umfaßt.

21. Verfahren zur Produktion des Proteins, das von einer die Aminosäuresequenz des Bordetella pertussis-Toxins codierenden Sequenz codiert wird, die eine vollständige oder verkürzte, die Aminosäuresequenz der S1-Untereinheit des B. pertussis-Toxins codierende Sequenz enthält, mit der Maßgabe, daß (a) die die S1-Untereinheit codierende Sequenz eine Modifikation des Tryptophanrestes an der Aminosäureposition 26 (nachstehend "Trp-26") enthält, und (b) die Trp-26-Modifikation eine im wesentlichen inaktivierte S1-Enzymaktivität bei dem Protein zur Folge hat, das von der die Aminosäuresequenz des B. pertussis-Toxin-Operons codierenden Sequenz codiert wird, wobei aber das Protein die Fähigkeit behalten hat, von Antikörpern gegen das Pertussis-Toxin erkannt zu werden, wobei ein solches Verfahren die Züchtung einer mit einem rekombinanten DNA-Plasmid, das die codierende Sequenz der rekombinanten DNA nach Anspruch 1 umfaßt, transformierten Wirtszelle in geeignetem Kulturmedium, und die Isolierung des so produzierten Proteins umfaßt.

22. Verfahren zur Produktion des Proteins, das von einer die Aminosäuresequenz des Bordetella pertussis-Toxins codierenden Sequenz codiert wird, enthaltend eine vollständige oder verkürzte, die Aminosäuresequenz der 51-Untereinheit des B. pertussis-Toxins codierende Sequenz, mit der Maßgabe, daß (a) die die S1-Untereinheit codierende Sequenz eine Modifikation des Tryptophanrestes an der Aminosäureposition 26 (nachstehend "Trp-26") enthält, und (b) die Trp-26-Modifikation eine im wesentlichen inaktivierte S1-Enzymaktivität bei dem Protein zur Folge hat, das von der die Aminosäuresequenz des B. pertussis-Toxin-Operons codierenden Sequenz codiert wird, wobei aber das Protein die Fähigkeit behalten hat, von Antikörpern gegen das Pertussis-Toxin erkannt zu werden, wobei ein solches Verfahren die folgenden Schritte umfaßt: (i) Behandlung einer die Aminosäuresequenz des Bordetella pertussis-Toxins codierenden Sequenz, die durch Isolierung aus nativer B. pertussis-DNA oder durch synthetische oder rekombinante DNA-Techniken hergestellt wurde und eine vollständige oder verkürzte, die Aminosäuresequenz der S1-Untereinheit des B. pertussis-Toxins codierende Sequenz enthält, mit der Maßgabe, daß die die S1-Untereinheit codierende Sequenz nicht bereits eine Modifikation des Tryptophanrestes an der Aminosäureposition 26 enthält, durch ortsgerichtete Mutagenese mit einem Tryptophan-modifizierenden chemischen Reagens oder durch Photooxidation zur Erzeugung einer Modifikation oder Deletion des Tryptophanrestes an der Aminosäureposition 26, Transformation einer gewünschten Wirtszelle mit einem Plasmid, das die erhaltene codierende Sequenz enthält, Züchtung des Wirts in einem geeigneten Kulturmedium und Isolierung des so produzierten Proteins, (ii) Substitution oder Deletion des Tryptophanrestes an der Position 26 der S1-Untereinheit einer die Aminosäuresequenz des Bordetella pertussis-Toxins codierenden Sequenz, die durch Isolierung aus nativer B. pertussis-DNA oder durch synthetische oder rekombinante DNA-Techniken hergestellt wurde und eine vollständige oder verkürzte, die Aminosäuresequenz der S1-Untereinheit des B. pertussis-Toxins codierende Sequenz enthält mit einer anderen Aminosäure, Transformation einer gewünschten Wirtszelle mit einem Plasmid, das die erhaltene codierende Sequenz enthält, Züchtung des Wirts in einem geeigneten Kulturmedium und Isolierung des so produzierten Proteins, oder (iii) Herstellung einer solchen codierenden Sequenz durch synthetische Verfahren, Transformation einer gewünschten Wirtszelle mit einem die resultierende codierende Sequenz enthaltenden Plasmid, Züchtung des Wirts in einem geeigneten Kulturmedium und Isolierung des so produzierten Proteins.

23. B. pertussis-Stamm, der ein inaktiviertes chromosomales Pertussis-Toxin-Gen enthält, das zusätzlich die codierende Sequenz nach Anspruch 1 umfaßt.

24. Impfstoff zur Stimulierung von Schutz gegen Keuchhusten, der eine immunprotektive und nicht-toxische Menge des Stamms nach Anspruch 23 umfaßt.

## Revendications

1. Séquence codante de toxine de Bordetella pertussis contenant une séquence codante sous-unitaire d'acides aminés, entière ou tronquée, de toxine de Bordetella pertussis, S1, à condition que (a) la séquence codante sous-unitaire S1 contienne une modification de résidu de tryptophane en position d'acide aminé 26 (ci-après « Trp-26 »), et que (b) la modification Trp-26 entraîne une substantielle inactivation de l'activité enzymatique de S1 dans la protéine encodée par la séquence codante de toxine de Bordetella pertussis, mais à condition qu'une telle protéine ait conservé la capacité d'être reconnue par les anticorps de toxine anti-pertussis.

2. Séquence codante de toxine de Bordetella pertussis selon la revendication 1, dans laquelle ladite séquence comprend la séquence codante entière d'acides aminés de l'opéron de toxine de Bordetella pertussis.

3. Séquence codante de toxine de Bordetella pertussis selon la revendication 1, dans laquelle ladite séquence codante comprend un opéron tronqué de toxine de Bordetella pertussis.

4. Séquence codante de toxine de Bordetella pertussis selon la revendication 1, dans laquelle ladite séquence codante comprend seulement la séquence codante sous-unitaire d'acides aminés S1.

5. Séquence codante de toxine de Bordetella pertussis selon la revendication 1, dans laquelle ladite séquence codante comprend seulement une portion tronquée de la séquence codante sous-unitaire d'acides aminés S1.

6. Séquence codante de toxine de Bordetella pertussis selon la revendication 5, dans laquelle ladite séquence codante comprend seulement les acides aminés 2 à 187 de la séquence codante sous-unitaire d'acides aminés S1.

7. Séquence codante de toxine de Bordetella pertussis selon la revendication 1, dans laquelle ladite séquence codante comprend en plus, au moins une autre séquence codante.

8. Séquence codante de toxine de Bordetella pertussis selon la revendication 7, qui a la séquence codante suivante :

ATG ACC ATG ATT ACG AAT TCG AGC TCG GTA CCC GGG GAT
(Met) Thr Met Ile Thr Asn Ser Ser Ser Val Pro Gly Asp
beta-galactosidase polylinker 2
S1

CCT CCC GCC ACC GTA......GTA GCG TCG ATC CTC TAG
Pro Pro Ala Thr Val........Val Ala Ser Ile Leu Stop
3 4 5 6 7 184 185 186 187 polylinker
S1

9. Séquence codante de toxine de Bordetella pertussis selon la revendication 1, dans laquelle ladite séquence codante comprend en plus, une modification d'un résidu supplémentaire d'acides aminés dans la séquence codante sous-unitaire S1, à condition que la protéine encodée par la séquence codante de toxine de Bordetella pertussis conserve la capacité d'être reconnue par les anticorps de toxine anti-pertussis, que la modification supplémentaire ne comprenne pas de délétion ou de substitution du résidu d'acide glutamique en position 129, et que, si le résidu de tryptophane en position 26 de la séquence codante sous-unitaire S1 est substitué par l'isoleucine, la modification supplémentaire ne comprenne pas la substitution du résidu d'acide aspartique en position d'acide aminé 11, par la sérine, la substitution du résidu d'arginine en position d'acide aminé 9, par la lysine, ou la glycine, la substitution du résidu de phénylalanine en position 50, par l'acide glutamique, la substitution du résidu d'arginine en position 13, par la leucine, la substitution du résidu d'acide aspartique en position 1, par l'acide glutamique, la substitution du résidu d'acide glutamique en position 39, par la glycine, la substitution du résidu de tyrosine en position 130, par la glycine, la substitution du résidu de glycine en position 86, par l'acide glutamique, la substitution du résidu d'isoleucine en position 88, par l'acide glutamique, la substitution du résidu de tyrosine en position 89, par la sérine, la substitution du résidu de tyrosine en position 8 par l'acide aspartique, ou la substitution du résidu de thréonine en position 53, par l'isoleucine.

10. Séquence codante de toxine de Bordetella pertussis selon la revendication 9, dans laquelle la modification supplémentaire se produit sur le résidu d'histidine en position d'acide aminé 35 de la séquence codante sous-unitaire S1 (His-35), le résidu d'arginine en position d'acide aminé 9 de la séquence codante sous-unitaire S1 (Arg-9), ou le résidu de sérine en position d'acide aminé 40 de la séquence codante sous-unitaire S1 (Ser-40).

11. Molécule d'ADN recombinant comprenant la séquence codante de toxine de Bordetella pertussis selon les revendications 1, 2, 3, 4, 5, 6, 7, 8, 9, ou 10.

12. Plasmide d'ADN recombinant comprenant la molécule d'ADN recombinant selon la revendication 11, en association opérationnelle avec des séquences convenables de contrôle d'expression.

13. Plasmide recombinant selon la revendication 12, dans lequel la modification du résidu de tryptophane en position 26 de la séquence codante sous-unitaire S1 est une mutation par délétion.

14. Plasmide recombinant selon la revendication 12, dans lequel la modification du résidu de tryptophane en position 26 de la séquence codante sous-unitaire S1 est une mutation par substitution.

15. Plasmide recombinant selon la revendication 14, dans lequel le résidu de tryptophane en position 26 est substitué par la thréonine.

16. Cellule hôte transformée par le plasmide selon la revendication 12.

17. Cellule hôte selon la revendication 16, qui est une souche de Bordetella pertussis dans laquelle l'opéron de toxine de pertussis est supprimé.

18. Protéine encodée par la séquence codante selon les revendications 1, 2, 3, 4, 5, 6, 7, 8, 9, et 10.

19. Vaccin pour la stimulation de la protection contre la coqueluche, dans lequel ledit vaccin comprend une quantité immunoprotectrice et non toxique, de la protéine encodée par la molécule d'ADN recombinant selon la revendication 1.

20. Procédé de production d'une cellule hôte transformée, qui comprend la transformation d'une cellule hôte désirée, par le plasmide d'ADN recombinant contenant la séquence codante selon la revendication 1.

21. Procédé de production de la protéine encodée par une séquence codante d'acides aminés de toxine de Bordetella pertussis contenant une séquence codante sous-unitaire d'acides aminés, entière ou tronquée, de toxine de Bordetella pertussis, S1, à condition que (a) la séquence codante sous-unitaire S1 contienne une modification de résidu de tryptophane en position d'acide aminé 26 (ci-après « Trp-26 »), et que (b) la modification Trp-26 entraîne une substantielle inactivation de l'activité enzymatique de S1 dans la protéine encodée par la séquence codante d'acides aminés de l'opéron de toxine de Bordetella pertussis, mais à condition qu'une telle protéine ait conservé la capacité d'être reconnue par les anticorps de toxine anti-pertussis, dans lequel ledit procédé comprend la culture d'une cellule hôte transformée par un plasmide d'ADN recombinant comportant la séquence codante d'ADN recombinant selon la revendication 1, dans un milieu de culture convenable, et l'isolement de la protéine ainsi produite.

22. Procédé de production de la protéine encodée par une séquence codante d'acides aminés de toxine de Bordetella pertussis contenant une séquence codante sous-unitaire entière ou tronquée d'acides aminés de toxine de Bordetella pertussis, S1, à condition que (a) la séquence codante sous-unitaire S1 contienne une modification de résidu de tryptophane en position d'acide aminé 26 (ci-après « Trp-26 »), et que (b) la modification Trp-26 entraîne une substantielle inactivation de l'activité enzymatique de S1 dans la protéine encodée par la séquence codante d'acides aminés de l'opéron de toxine de Bordetella pertussis, mais à condition qu'une telle protéine ait conservé la capacité d'être reconnue par les anticorps de toxine anti-pertussis, un tel procédé comprenant (i) le traitement d'une séquence codante d'acides aminés de toxine de Bordetella pertussis, préparée par isolement à partir d'ADN naturel de Bordetella pertussis, ou par des techniques de synthèse, ou d'ADN recombinant, contenant une séquence codante sous-unitaire entière ou tronquée d'acides aminés de toxine de Bordetella pertussis, S1, à condition que la séquence codante sous-unitaire S1 ne contienne pas déjà une modification de résidu de tryptophane en position d'acide aminé 26, par mutagenèse orientée vers le site, par un agent chimique ou une photo-oxydation modifiant le tryptophane, pour produire une modification ou une délétion du résidu de tryptophane en position d'acide aminé 26, et par transformation d'une cellule hôte désirée, à l'aide d'un plasmide contenant la séquence codante ainsi obtenue, et la culture d'un tel hôte dans un milieu de culture convenable, et l'isolement de la protéine ainsi produite, (ii) la substitution ou la délétion du résidu de tryptophane en position 26 de la séquence codante sous-unitaire d'acides aminés de toxine de Bordetella pertussis, S1, préparée par isolement à partir d'ADN naturel de Bordetella pertussis, ou par des techniques de synthèse ou d'ADN recombinant, contenant une séquence codante sous-unitaire entière ou tronquée d'acides aminés de toxine de Bordetella pertussis, S1, par un autre acide aminé, et la transformation d'une cellule hôte désirée, à l'aide d'un plasmide contenant la séquence codante ainsi obtenue, et la culture d'un tel hôte dans un milieu de culture convenable, et l'isolement de la protéine ainsi produite, ou (iii) la préparation d'une telle séquence codante par des méthodes de synthèse, et la transformation d'un hôte désiré à l'aide d'un plasmide contenant une séquence codante ainsi obtenue, et la culture d'un tel hôte dans un milieu de culture convenable, et l'isolement de la protéine ainsi produite.

23. Souche de Bordetella pertussis qui contient un gène de toxine de pertussis chromosomal inactivé, et qui comprend en plus, la séquence codante selon la revendication 1.

24. Vaccin pour la stimulation de la protection contre la coqueluche, ledit vaccin comprenant une quantité immunoprotectrice et non toxique de la souche selon la revendication 23.
